# EUROPEAN PATENT SPECIFICATION

(11) **EP 2 696 820 B1**
(45) Date of publication and mention of the grant of the patent: **03.06.2015**
(21) Application number: 12720261.2
(22) Date of filing: 02.04.2012
(51) Int. Cl.: A61F 5/56, A61F 5/055

(54) **CHIN AND NECK SUPPORT DEVICE**
KINN- UND NACKENSTÜTZEINRICHTUNG
DISPOSITIF DE SOUTIEN DE MENTON ET DE COU

(30) Priority: 15.04.2011 GB 201106415
(43) Date of publication of application: 19.02.2014
(73) Proprietor: Hassan, Muthana T., Hastings, East Sussex TN34 2HA (GB)
(72) Inventor: Hassan, Muthana T., Hastings, East Sussex TN34 2HA (GB)
(74) Representative: Brookes Batchellor LLP
(86) International application number: PCT/GB2012/050739
(87) International publication number: WO 2012/140408

(56) References cited:
- WO-A1-2010/068251
- WO-A2-2008/083193
- US-A- 3 776 224

## Description

Background:
This invention relates to a chin and neck support device. In particular, the present invention relates to a chin and neck support device to reduce or eliminate snoring and
sleep apnoea provided there is no nasal airway obstruction or pathology. WO-A-2008/083193 is regarded as the closest prior art.

Snoring is a loud and vibratory noise produced by turbulent airflow caused by restriction of air passages through partially obstructed airways as a result of lower jaw drop, the chin resting on the chest and the tongue falling backwards onto the soft palate and the pharynx. This is due to muscle relaxation of the throat, back of the tongue, lower jaw and neck. Snoring occurs in almost all cases owing to neck flexion, breathing through the mouth and dropping of the lower jaw during sleep on the back or even on the sides.

Snoring can also occur when sleeping in a sitting position. Examples are people who sit for long hours during travel and the elderly people who sleep in a sitting position. Many cases of heavy snoring can lead to sleep apnoea which is a sleep disorder characterised by pauses in breathing which can last from a few seconds to a minute and can occur from five to thirty times an hour. Apnoea associated with snoring is most commonly obstructive in nature. In sleep apnoea, the breathing pauses and is accompanied by a pattern of snoring.

The principles of carrying out chin, lower jaw elevation and head extension are demonstrated when respiratory support is applied to unconscious people or after general anaesthesia to maintain patent (unobstructed) airway passages. When snoring occurs during sleep, the lower jaw, base of tongue, back of throat and neck are relaxed as they are under general anaesthesia. The present invention aims to overcome the problem of relaxation of the lower jaw, base of tongue, throat and neck, provided there is no nasal airway obstruction or pathology. The device also provides neck support.

### Statement of invention:

The present invention relates to a device for chin and neck support to reduce or eliminate snoring. According to the present invention, there is provided a chin and neck support device comprising: a chin support; a chest support; an adjustable swivel joint for mounting the chest support; an adjustable telescopic rod for connecting the chin support and the chest support; a U-shaped frame; and a neck strap securable to the U-shaped frame, wherein, the U-shaped frame is a malleable frame which, in use, is connected to the telescopic rod and prevents contact between the device and an anterior aspect of a wearer's neck.

Preferably, the chin support is concave in shape with a padded chin contact surface. Suitably, the chin support is made from a malleable material.

Ideally, the chest support, in use, rests on the upper sternum (Manubrium) and is rigid, flat and has a padded area. Conveniently, the padded area is of 5-10 cm in length to spread the load.

In one embodiment, the adjustable telescopic rod has a length of 5 and 14 cm (variable) to keep the head in an erect position, in use, and prevent neck flexion during sleep or whilst in a sitting position.

Preferably the neck strap is securable at a front part of the U shaped frame.

### Introduction to Drawings:

An example of the invention will now be described by referring to the accompanying drawings in which:

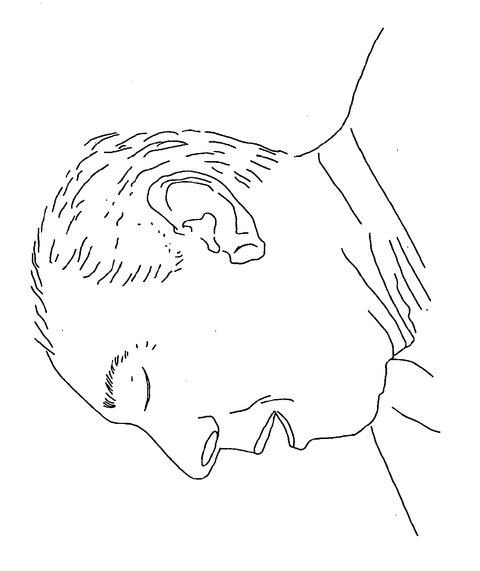

Figure 1 shows a person sleeping on the side with mouth open and lower jaw falling back almost onto the chest, demonstrating a snoring position.

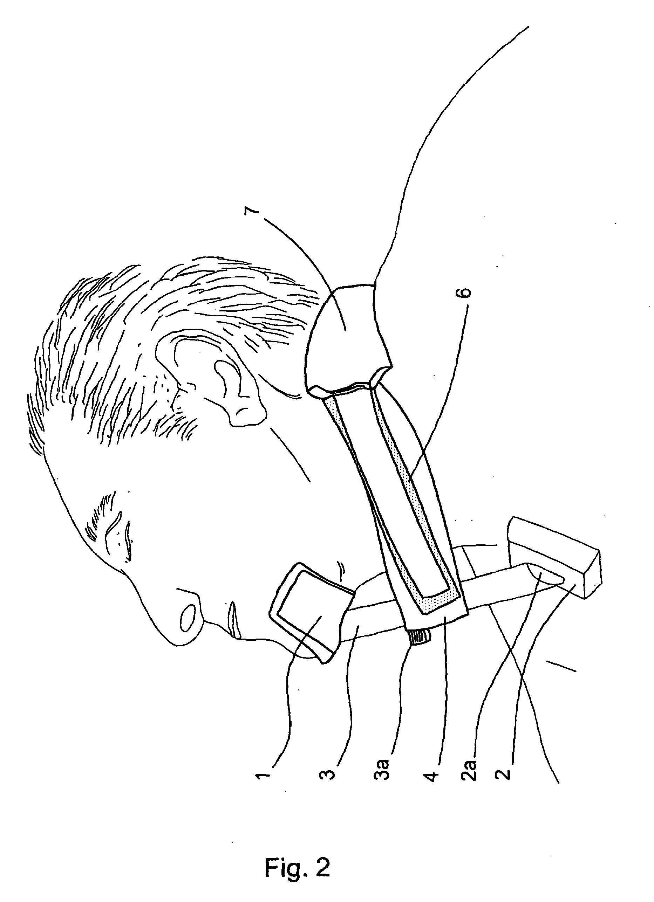

Figure 2 shows a user asleep on the side wearing the device, demonstrating the chin lift and the lower jaw supported upwards, maintaining a closed mouth with unobstructed air passages.

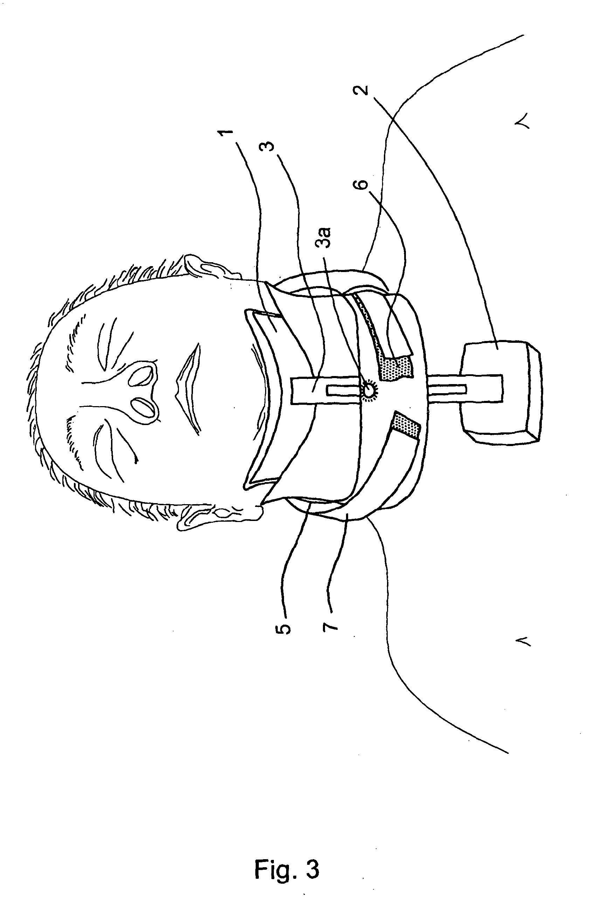

Figure 3 shows the user asleep on his back whilst wearing the device, demonstrating the chin lift; the lower jaw supported upwards, the mouth closed and maintaining the head in an erect position with unobstructed air passages.

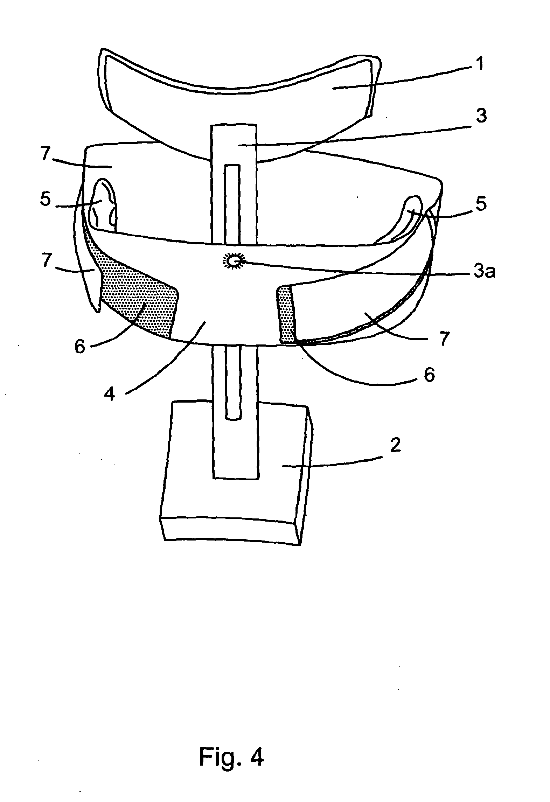

Figure 4 shows the device from the front (or anterior view).

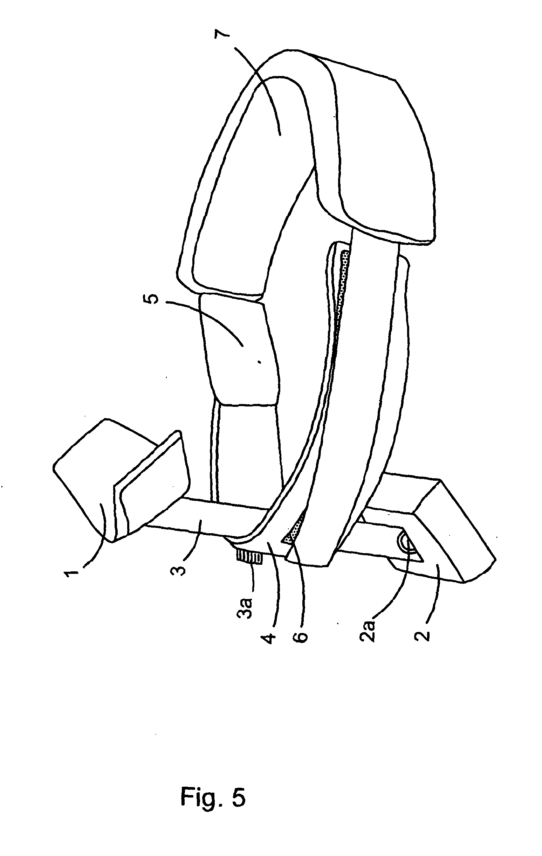

Figure 5 shows side view of the device.

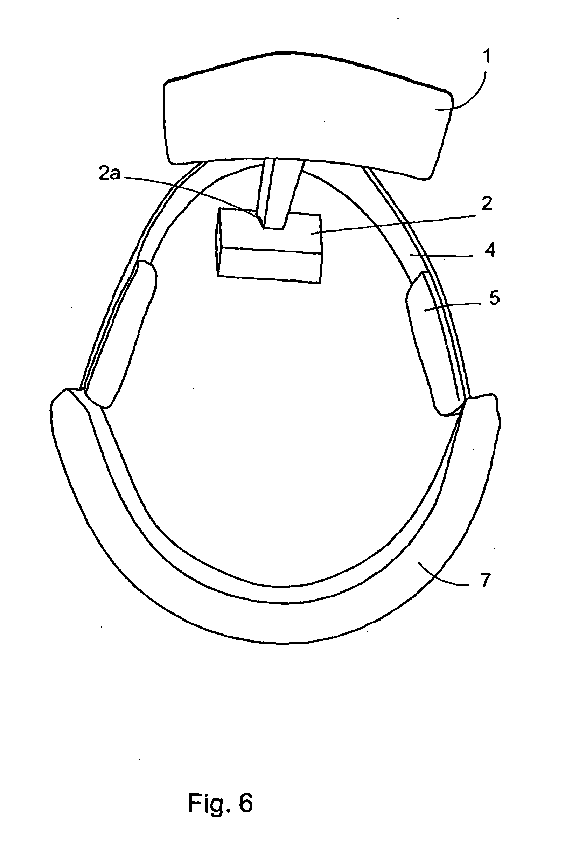

Figure 6 shows the device from above.

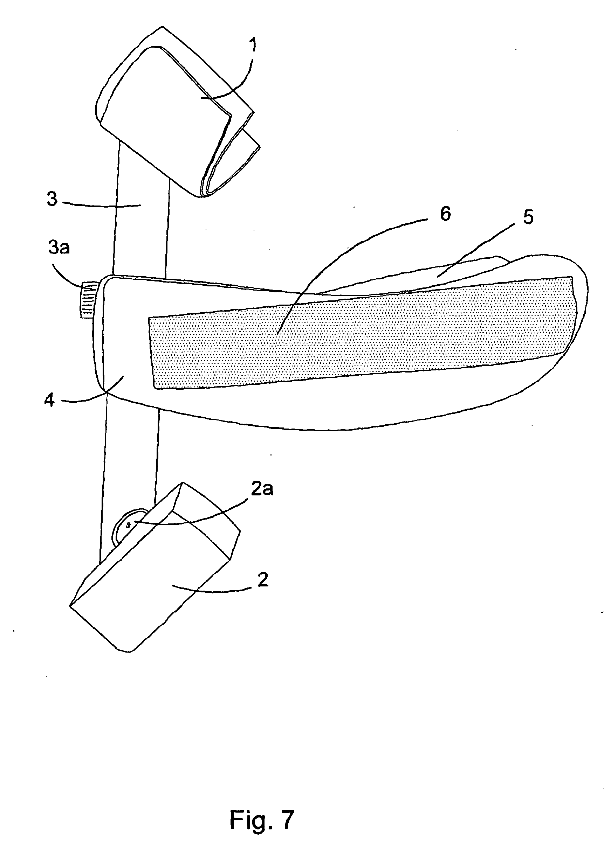

Figure 7 shows the device from the side without the neck strap.

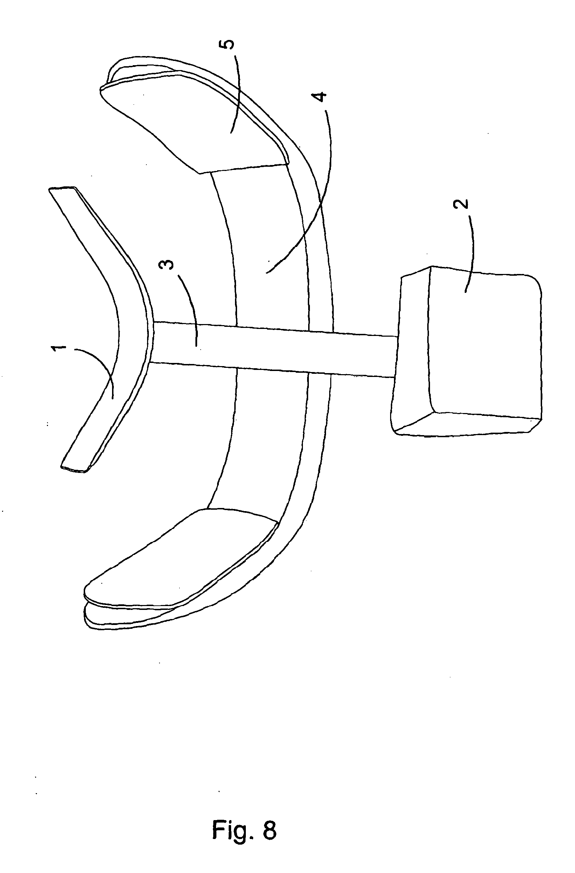

Figure 8 shows the device from behind (Rear view) without the neck strap.

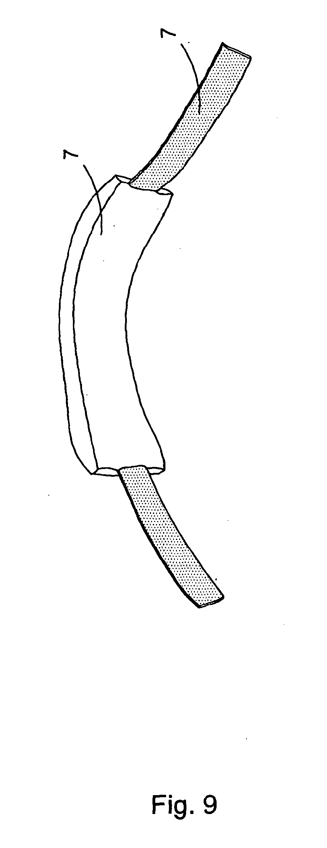

Figure 9 shows the neck strap.

### Detailed Description

The principles of the chin and neck support device are designed to provide direct lower jaw support, keep the head in an erect position and prevent flexion and rotational movements of the neck, but also allow full extension and side movements of the neck when worn in any position during sleep except the prone position and when worn in a sitting position.

A chin support (1) is concave in shape and is made from malleable material to accommodate the user's chin shape, with a padded surface for comfort. A chest support (2) is connected to the upper end of an adjustable telescopic Rod (3).

Chest support (2) is flat, padded and rigid; it can be in any suitable shape. It has a padded surface for comfort. Chest support (2), in use, rests on the upper part of the sternum (Manubrium). An adjustable swivel joint (2a) is attached to its base to allow adjustment of the chest support (2) according to the user's chest shape when in use. The chest support (2) is connected to the lower end of the telescopic rod (3) by an adjustable swivel joint (2a) on chest support (2). The swivel joint (2a) allows for the necessary adjustment of the angle between the chest support (2) and the adjustable telescopic rod (3) to ensure support for the lower jaw and to keep the head in an erect position.

The adjustable telescopic rigid rod (3), which can be replaced by sliding surface, is square or rounded in shape with a knob (3a) for adjustment .The adjustable telescopic rod (3) is not in contact with the neck. The adjustable telescopic rod (3) is connected to the chin and chest supports, providing support to the lower jaw and keeping the mouth closed and the head in an erect position. The length of the rod can be adjusted according to the user's need and shape of their lower jaw and their chest.

The combination of adjustable telescopic rod (3) and adjustable swivel joint (2a) on the chest support (2), when worn, will provide universal use for different neck sizes and genders.

An adjustable "U" shaped frame (4) is connected to a front part of the adjustable telescopic rod. The U shaped frame is made of a malleable material (which can be adjusted according to the diameter of the user's neck). Both arms of the "U" shaped frame (4), in use, are parallel to the sides of the neck, with both inside surfaces (about 3-6 cm), being in superficial contact with the sides of the neck, without causing pressure or discomfort, when worn. The last 3-6 cm of the inside surface of both ends (5), of the "U" shaped frame, are padded for comfort. The last 5cm-7cm of both arms of the "U" shaped frame (4) are at about a ten degree angle, pointing upwards so that both arms of the U shaped frame(4), in use, don't rest on the clavicles on the sides of the neck and to allow secured attachment and support to the neck strap (7). A band of Velcro, or another means of attachment, is fixed onto the outer side surfaces (6) of the arms (4) of the U shaped frame and on the inside surfaces of the padded neck strap (7) when worn.

The "U" shaped frame (4) gives support and fixes the telescopic rod (3) in a central position, in order to keep the chin support (1) and the chest support (2) securely in place when worn during sleep or in a sitting position.

The "U" shaped frame(4),in use, is not in contact with the front of the neck and is about 2-5cm away from the front of the neck, allowing space and access to the neck, air circulation around the neck and no interference with swallowing or breathing. This prevents the effect of claustrophobia when wearing the device during sleep or in a sitting position.

A padded neck strap (7) is securable at the front of the U shaped frame (4) when in use. To hold the device in position under the chin and on the chest, padded neck strap (7) with Velcro (or another means of attachment on the inside surfaces on both ends) is attached to the Velcro (or another means of attachment) on the outer surface of both arms (6) of the "U" shaped frame (4). The attachment is adjustable and securable according to the size of the user's neck when in use.

There is easy access to the padded neck straps (7), in use, from the either side of the front of the neck, facilitating fast fastening and removal.

The pressure holding the device in place, when worn, is comfortably distributed through the padded neck strap (7) across the back of the neck.

The Velcro fixation (or another means of attachment) on both arms of the "U" shaped frame (6) can readily be detached allowing for rapid removal of the device.

This invention is designed to solve a problem which is prevalent and often difficult to treat, provided there is no nasal airway obstruction or pathology.

The device is not a collar, the aspect of this device which defies classification as a collar:
1. It specifically restricts neck flexion, while allowing almost full neck extension, lateral tilting and side movements of the neck.
2. The device minimises contact with the skin generally, and completely avoids skin contact over the anterior aspect of neck.
3. It allows unimpeded swallowing, minimally impeded air circulation and sweating, with optimisation of wearer comfort.
4. The critical function of prevention of jaw drop and neck flexion can be optimised by specific adjustment of the anterior vertical dimension of the device, allowing customisation to any adult physique.

Advantages:
1. This device treats snoring and sleep apnoea provided there is no nasal airway obstruction or pathology.
2. This device allows the support to be delivered flexibly, consistently and tolerably, permitting the wearer to sleep in an undisturbed manner.
3. Features of the device include its construction, adjustability, , its comfort and its easy removability
4. This device is non invasive.
5. This device doesn't interfere with breathing or swallowing because the device is not in contact with front aspect of the neck.
6. The device has very minimal movements during breathing because the upper part of sternum, on which this device is supported, has very minimum movements during respiration.
7. This device is not in contact with the front of the neck. This allows free air circulation and minimisation of uncomfortable sweating.
8. This device prevents flexion and rotational movements of the neck but allows full extension and sideways movements of the neck when worn during sleep or in a sitting position because of the effect of the adjustable telescopic rod.
9. This device offers ease of application and removal from either front side of the neck because of the removable neck strap in use in front of the U shaped frame.
10. This device will keep the mouth substantially closed, support the lower jaw and keeps the head in an erect position during sleep or in a sitting position because of the support of the telescopic rod.
11. The Swivel joint on the padded chest support, allows adjustment according to the shape of the individual's chin and chest, maximising support potential.
12. The telescopic rod (or sliding surface) between chin support and chest support, in addition to a swivel joint on the chest pad, allow adjustment of the length and the angle of the rod with respect to the chest support, necessary to maximise device function, irrespective of variation in user's anatomy. It maintains the head in an erect position, with the mouth substantially closed and giving support to the lower jaw during sleep on either side or in the supine position when the device is worn.
13. A padded strap behind the neck, in use ,is attached to an affixed Velcro, or other means of attachment, on both arms of the "U" shaped frame securing the device is worn, in a comfortable position. It is an adjustable neck strap.
14. Both arms of the Malleable or bendable "U" shaped frame are adjustable according to the diameter of the user's neck when in use.
15. This device is comfortable to wear, permitting normal sleep when worn in a lying down or in a sitting position because the telescopic rod prevents drop of the lower jaw, and the device is not in contact with the front aspect of the neck..
16. This device can be worn on a daily basis as a neck support in a sitting position for people with neck problems, and for people who sleep in a sitting position because of support to the lower jaw and prevention of neck flexion.
17. This device can be worn by passengers to support their neck and will facilitate sleep in the sitting position during long journeys using all types of transport e.g. aeroplanes, trains and buses because of the support to the lower jaw and the prevention of neck flexion.
18. This device can be used for patients in recovery wards after having general anaesthetics because the device helps to maintain patent (unobstructed) airways by supporting the lower jaw and preventing the neck flexion until full recovery is achieved.
19. It is light in weight so it is easy to handle and to wear.
20. This device will bring relief for many people who suffer from snoring, which causes constant disruption and irritation to their sleep and that of their families and loved ones.
21. The Relatively simple design facilitates assembly and optimal function.

## Claims

1. A chin and neck support device comprising;
a chin support (1);
a chest support (2);
an adjustable swivel joint (3a) for mounting the chest support (2); and
an adjustable telescopic rod (3) for connecting the chin support and the chest support;
**characterised in that** the support device further comprises;
a "U" shaped frame (4); and
a neck strap (7) securable to the U-shaped frame (4);
wherein the U-shaped frame (4) is a malleable frame which, in use, is connected to the telescopic rod (3) and prevents contact between the device and an anterior aspect of a wearer's neck.

2. A chin and neck support device according to claim 1, in which the chin support (1) is concave in shape with a padded chin contact surface.

3. A chin and neck support device according to claim 1 or claim 2, in which the chin support (1) is made from a malleable material.

4. A chin and neck support device according to any one of claims 1 to 3, in which the chest pad (2), in use, rests on the upper sternum (Manubrium) and is rigid, flat and has a padded area of 5-10 cm length to spread the load.

5. A chin and neck support device according to any one of the preceding claims wherein the adjustable telescopic rod (3) has a length of between 5 and 14 cm to keep the head in an erect position during use.

6. A chin and neck support device according to any one of the preceding claims, wherein the neck strap (7) is securable at a front part of the U-shaped frame (4).

## Patentansprüche

1. Kinn- und Nackenstützvorrichtung, umfassend:
eine Kinnstütze (1);
eine Bruststütze (2);
ein verstellbares Drehgelenk (3a) zum Aufstellen der Bruststütze (2); und
eine verstellbare Teleskopstange (3) zum Verbinden der Kinnstütze mit der Bruststütze;
**dadurch gekennzeichnet, dass** die Stützvorrichtung ferner Folgendes umfasst:
ein U-förmiges Gestell (4); und
ein Nackenband (7), das an dem U-förmigen Gestell (4) befestigt werden kann;
wobei das U-förmige Gestell (4) ein verformbares Gestell ist, das im Gebrauch mit der Teleskopstange (3) verbunden ist und einen Kontakt zwischen der Vorrichtung und einer anterioren Seite des Nackens eines Trägers verhindert.

2. Kinn- und Nackenstützvorrichtung nach Anspruch 1, wobei die Kinnstütze (1) mit einer gepolsterten Kinnkontaktfläche konkav geformt ist.

3. Kinn- und Nackenstützvorrichtung nach Anspruch 1 oder 2, wobei die Kinnstütze (1) aus einem formbaren Material hergestellt ist.

4. Kinn- und Nackenstützvorrichtung nach einem der Ansprüche 1 bis 3, wobei das Brustpolster (2) im Gebrauch auf dem oberen Brustbein (Manubrium) ruht und starr und flach ist und eine 5 bis 10 cm lange gepolsterte Fläche zum Verteilen der Belastung aufweist.

5. Kinn- und Nackenstützvorrichtung nach einem der vorhergehenden Ansprüche, wobei die verstellbare Teleskopstange (3) eine Länge zwischen 5 und 14 cm aufweist, um den Kopf im Gebrauch in einer aufrechten Position zu halten.

6. Kinn- und Nackenstützvorrichtung nach einem der vorhergehenden Ansprüche, wobei das Nackenband (7) an einem vorderen Teil des U-förmigen Gestells (4) befestigt werden kann.

## Revendications

1. Dispositif de support de menton et de cou comprenant,
un support de menton (1),
un support de cou (2),
un raccord articulé ajustable (3a) destiné à monter le support de cou (2), et
une tige télescopique ajustable (3) destinée à connecter le support de menton et le support de cou,
**caractérisé en ce que** le dispositif de support comprend en outre,
une structure en forme de U (4), et
une bande cervicale (7) pouvant être fixée à la structure en forme de U (4),
dans lequel la structure en forme de U (4) est une structure malléable qui, en utilisation, est connectée à la tige télescopique (3) et empêche un contact entre le dispositif et un aspect antérieur d'une région cervicale d'un porteur.

2. Dispositif de support de menton et de cou selon la revendication 1, dans lequel le support de menton (1) est de forme concave avec une surface de contact avec le menton rembourrée.

3. Dispositif de support de menton et de cou selon la revendication 1 ou la revendication 2, dans lequel le support de menton (1) est réalisé à partir d'un matériau malléable.

4. Dispositif de support de menton et de cou selon l'une quelconque des revendications 1 à 3, dans lequel le coussin de cou (2), en utilisation, repose sur le sternum supérieur (manubrium) et est rigide, plat et possède une zone rembourrée d'une longueur de 5 à 10 cm pour répartir la charge.

5. Dispositif de support de menton et de cou selon l'une quelconque des revendications précédentes dans lequel la tige télescopique ajustable (3) est d'une longueur entre 5 et 14 cm pour maintenir la tête dans une position redressée au cours de l'utilisation.

6. Dispositif de support de menton et de cou selon l'une quelconque des revendications précédentes, dans lequel la bande cervicale (7) peut être fixée au niveau d'une partie avant de la structure en forme de U (4).
